# FASCICULE DE BREVET EUROPEEN

(11) **EP 0 831 090 B1**
(45) Date de publication et mention de la délivrance du brevet: **12.04.2000**
(21) Numéro de dépôt: 97402175.0
(22) Date de dépôt: 19.09.1997
(51) Int. Cl.: C07D 401/06, C07D 401/14, C07D 413/06, C07D 473/00, C07D 409/06, C07D 407/06, A61K 31/445, C07D 405/06, C07D 417/06

(54) **Amines cycliques N-substituées, leur procédé de préparation et les compositions pharmaceutiques les renfermant**
N-substituierte zyklische Amine, ihre Herstellungsverfahren und sie enthaltende pharmazeutische Zusammensetzungen
N-substituted cyclic amines, process for their preparation and pharmaceutical compositions containing them

(30) Priorité: 20.09.1996 FR 9611501
(43) Date de publication de la demande: 25.03.1998
(73) Titulaire: ADIR ET COMPAGNIE, 92415 Courbevoie Cédex (FR)
(72) Inventeur: Dhainaut, Alain, 78400 Chatou (FR); Tizot, André, 91370 Verrieres Le Buisson (FR); Canet, Emmanuel, 75014 Paris (FR); Lonchampt, Michel, Clos Dericbourg, 94150 Chevilly La Rue (FR)
(74) Mandataire: Reverbori, Marcelle

(56) Documents cités:
- EP-A- 0 718 286
- EP-A- 0 745 591
- WO-A-94/25437
- FR-M- 6 850

## Description

La présente invention a pour objet de nouvelles amines cycliques N-substituées, leur procédé de préparation et les compositions pharmaceutiques les renfermant.

Ces dites amines sont des inhibiteurs de phosphodiestérases du groupe 4 et de ce fait présentent des applications thérapeutiques particulièrement intéressantes.

En effet, les fonctions de la plupart des tissus organiques sont modulées par des substances endogènes (hormones, neurotransmetteurs, autacoïdes) ou exogènes. Pour certaines de ces substances, l'effet biologique est relayé au niveau intracellulaire par des effecteurs enzymatiques tels que l'adénylate cyclase ou la guanylate cyclase. la stimulation de ces enzymes responsables de la synthèse de nucléotides cycliques tels que l'adenosine-3',5'-monophospate cyclique (AMPc) et la guanosine-3',5'-monophosphate cyclique (GMPc) entraîne une élévation du taux intracellulaire de ces seconds messagers impliqués dans la régulation de nombreuses fonctions biologiques (E. W. SUTHERLAND et T. W. RALL, Pharmacol. Rev., Vol. 12, p 265, 1960).

La dégradation des nucléotides cycliques est assurée par une famille d'enzymes, appelées phosphodiestérases (PDE), classées actuellement en 7 groupes. La reconnaissance d'isoformes différentes à l'intérieur de chacun de ces groupes, et de la distribution tissu- ou cellule-spécifique de certaines isoformes, a stimulé la recherche d'inhibiteurs de plus en plus spécifiques de tel ou tel type d'isoenzyme (J. A. BEAVO, Physiological Rev., Vol 75, n° 4, pp 725-749, 1995). Parmi les différentes familles de PDE, la PDE4 a été identifiée dans de très nombreux tissus ou cellules comme le cerveau, le coeur, l'endothélium vasculaire, le muscle lisse vasculaire et trachéobronchique et les cellules hématopoiétiques. L'inhibition des phosphodiestérases ralentit l'hydrolyse des nucléotides cycliques et entraîne une augmentation de la teneur en AMPc et/ou GMPc.

Les inhibiteurs de PDE4, responsables d'une augmentation des taux d'AMPc, possèdent des activités anti-inflammatoires et des effets relaxants sur le muscle lisse trachéobronchique d'où leur intérêt thérapeutique dans le domaine de la pathologie respiratoire ou des pathologies associées à un processus inflammatoire (M. N. PALFREYMAN, Drugs of the Future, Vol. 20, n° 8, pp 793-804, 1995 ; J. P. BARNES, Eur. Respir. J., Vol. 8, pp 457-462, 1995 ; S.B. CHRISTENSEN et T. J. TORPHY, Annual Reports in Medicinal Chemistry, Vol. 29, pp 185-194, 1994, Academic Press).

La présente invention concerne particulièrement les amines cycliques N-substituées de formule I : 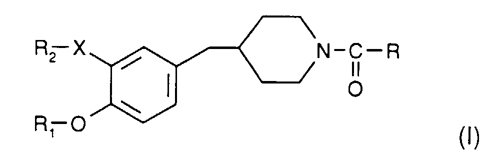 dans laquelle :
- X est choisi parmi -CH=, -CH₂- et un atome d'oxygène ;
- R₁ représente un radical choisi parmi le groupe constitué par les radicaux (C₁-C₆)alkyle, en chaîne droite et ramifiée, non substitués et substitués par un et plusieurs atomes d'halogène (tels que par exemple les atomes de fluor et chlore) ;
- R₂ représente un radical choisi parmi le groupe constitué par :
   a) les radicaux hydrocarbonés monocycliques saturés et non-saturés contenant de 3 à 6 atomes de carbone non substitués et ces mêmes radicaux substitués par un et plusieurs substituants choisis parmi le groupe constitué par : les atomes d'halogène (tels que par exemple les atomes de fluor et chlore) et le radical hydroxy ;
   b) les radicaux hydrocarbonés polycycliques saturés (tel que par exemple le radical bicyclo[2,2,1]heptyle et le radical adamantyle) et non-saturés contenant de 7 à 10 atomes de carbone non-substitués et ces mêmes radicaux substitués par un et plusieurs substituants choisis parmi le groupe constitué par : les atomes d'halogène (tels que par exemple les atomes de fluor et chlore) et le radical hydroxy ;
   c) les radicaux (C₁-C₁₃) hydrocarbonés saturés et non saturés en chaîne droite et en chaîne ramifiée, non-substitués et substitués par un et plusieurs substituants choisis parmi les atomes d'halogène (tels que par exemple les atomes de fluor et chlore) et le radical hydroxy ; et
   d) les radicaux (C₁-C₁₃)hydrocarbonés définis au paragraphe c) ci-dessus, substitués de plus par un et plusieurs radicaux choisis parmi le groupe constitué par :
      - le radical phényle non substitué et les radicaux phényles substitués par un et plusieurs substituants choisis parmi le groupe constitué par les atomes d'halogène (tels que, par exemple, les atomes de fluor et chlore) et le radical hydroxy ;
      - les radicaux hydrocarbonés monocycliques saturés et non-saturés contenant de 3 à 6 atomes de carbone non substitués et ces mêmes radicaux substitués par un et plusieurs substituants choisis parmi le groupe constitué par : les atomes d'halogène (tels que par exemple les atomes de fluor et chlore) et le radical hydroxy ;
      - les radicaux hydrocarbonés polycycliques, saturés (tel que par exemple le radical bicyclo[2,2,1]heptyle et le radical adamantyle) et non-saturés, contenant de 7 à 10 atomes de carbone non-substitués et ces mêmes radicaux substitués par un et plusieurs substituants choisis parmi le groupe constitué par : les atomes d'halogène (tels que par exemple les aotmes de fluor et chlore) et le radical hydroxy ;
- R est choisi parmi le groupe constitué par
   - les radicaux phényle, biphénylyle et naphtyle ;
   - les radicaux aromatiques, comportant 5 chaînons et contenant de 1 à 4 hétéroatomes (identiques et différents), comme par exemple les radicaux furyle, thiényle, pyrrolyle, imidazolyle, pyrazolyle, thiazolyle, isothiazolyle, oxazolyle, isoxazolyle, oxadiazolyle, triazolyle et le tétrazolyle ;
   - les radicaux aromatiques, comportant 6 chaînons et contenant 1 à 3 atomes d'azote, comme par exemple les radicaux pyridyle, pyrimidinyle, pyrazinyle, pyridazinyle et triazinyle ;
   - les radicaux aromatiques bicycliques saturés et non- saturés, comportant de 9 à 10 atomes dont 1 à 4 hétéroatomes (identiques et différents), tels que par exemple les radicaux indolyle, indolinyle, benzofuranyle, benzothiényle, benzimidazolyle, benzopyranyle, benzopyrazolyle, benzotriazolyle, benzoxazolyle, benzisoxazolyle, chromanyle, chroményle, quinolyle, dihydro et tétrahydroquinolyle, isoquinolyle, dihydro et tétrahydroisoquinolyle, quinazolinyle, quinoxalinyle, phtalazinyle, cinnolinyle, naphtyridinyle, pyridopyridyle, ptéridinyle et purinyle ;
   - les radicaux aromatiques bicycliques partiellement saturés ci-dessus définis contenant une et plusieurs fonctions carbonylées ;
      et
   - chacun des groupements précédemment définis entrant dans la signification de R, substitués par 1 à 3 substituants choisis parmi les atomes d'halogène, les radicaux R₁, -O-CO-R₁, (R₁ ayant la signification précédemment définie), CN, NO₂, OR₃, SR₃, COR₃, COOR₃, NR₃R₄, NCOR₃, CONR₃R₄, SO₂NR₃R₄ dans lesquels, R₃ et R₄ identiques ou différents représentent chacun un atome d'hydrogène ou R₁ tel que précédemment défini.

Les composés de formule I peuvent exister sous forme d'isomères géométriques et/ou d'isomères optiques, qui isolément ou en combinaison, font également partie de la présente invention.

Selon les valeurs de R notamment, certains composés de formule I peuvent aussi exister sous forme de sels d'addition avec des acides ou des bases pharmaceutiquement acceptables, lesquels sels physiologiquement acceptables sont aussi inclus dans la présente invention.

L'état antérieur de la technique la plus proche de la présente invention est illustré notamment par la demande de brevet WO 94/25437 A₁ qui mentionne des phénylpipéridines N-acylées comme agents bronchodilatateurs et antiinflammatoires.

La présente invention a également pour objet le procédé de préparation des composés de formule I :
caractérisé en ce que l'on fait réagir :
- l'amine secondaire cyclique de formule II : 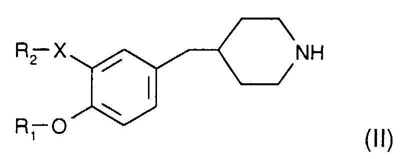 dans laquelle : X, R₁ et R₂ ont les significations précédemment définies, avec
- le composé carbonylé de formule III : 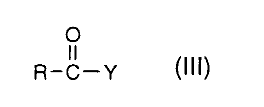 dans laquelle :
   - R a la signification précédemment définie et
   - Y représente un radical hydroxy ou un atome de chlore

Lorsque Y représente un atome de chlore, il est particulièrement intéressant d'effectuer la condensation selon les méthodes connues en présence d'une amine tertiaire comme la triéthylamine ou la 4-diméthylaminopyridine, ou en présence d'une base minérale telle que la soude, la potasse, le carbonate de sodium ou de potassium faisant office d'accepteur de l'hydracide formé, dans un solvant aprotique apolaire tel que le dichlorométhane, l'éther éthylique ou le tétrahydrofurane, ou encore dans un solvant aprotique polaire tel que le diméthylformamide et ceci dans une gamme de température de 0 à 50°C, et en maintenant le temps de contact des réactifs de 1 à 20 heures.

Lorsque Y représente un radical hydroxy, il est intéressant d'utiliser les méthodes de couplage peptidique (M. BODANSZKY et A. BODANSZKY, The Practice of peptides Synthesis, Springer-verlag, 1984) et plus particulièrement celles utilisant le dicyclohexylcarbodiimide (DCC) ou un de ses dérivés. La liaison amide peut aussi être réalisée en présence de tétrafluoroborate de 2-(1H-benzotriazole-1-yl)-1,1,3,3-tétraméthyluronium (TBTU) auquel on peut ajouter un activateur tel que l'hydroxybenzotriazole (HOBT) selon les méthodes décrites (M. S. BERNATOWICZ et al., Tetrahedron Lett., Vol. 30, p 4645, 1989 ; A. G. BECK-SICKINGER, H. DURR et G. JUNG, Pept. Res., Vol. 4, p 88, 1991 ; G. E. REID et R. J. SIMPSON, Anal. Biochem., Vol. 200, p 301, 1992 ; C. G. FIELS, D. H. LLOYD, R. L. MACDONNALD, K. M. OTTESON et R. L. NOBLE, Pept. Res., Vol. 4, p 95, 1991) , dans un solvant aprotique apolaire tel que l'éther éthylique, le tétrahydrofurane ou le dichlorométhane dans une gamme de température de 0 à 50 °C selon le solvant choisi. Il peut être aussi particulièrement intéressant de former la liaison amide en présence d'anhydride propylphosphonique et de N-éthylmorpholine selon la méthode décrite par H. WISSMANN et H. J. KLEINER, Angew. Chem. Int. Ed., Vol 19, pp 133-134, 1980. Lorsque la méthode employant l'anhydride propylphosphonique est choisie, il est particulièrement intéressant d'utiliser le diméthylformamide comme solvant et ceci dans une gamme de température de 0 à 100°C. Selon l'acide et la méthode choisis, le temps de contact optimal peut varier de 1 à 20 heures dans les gammes de température précédemment définies.

Les matières premières de formule Il ont été préparées par hydrolyse des composés correspondants de formule IV : 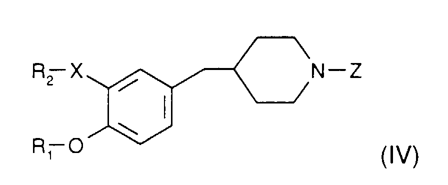 dans laquelle :
- X, R₁ et R₂ ont les significations précédemment définies, et
- Z représente un groupe acyle ou alkoxycarbonyle.

Les composés de formule IV peuvent eux-mêmes être préparés selon le schéma réactionnel suivant dans lequel, sauf mention restrictive précisée, X, R₁, R₂ et Z ont les significations précédemment définies :

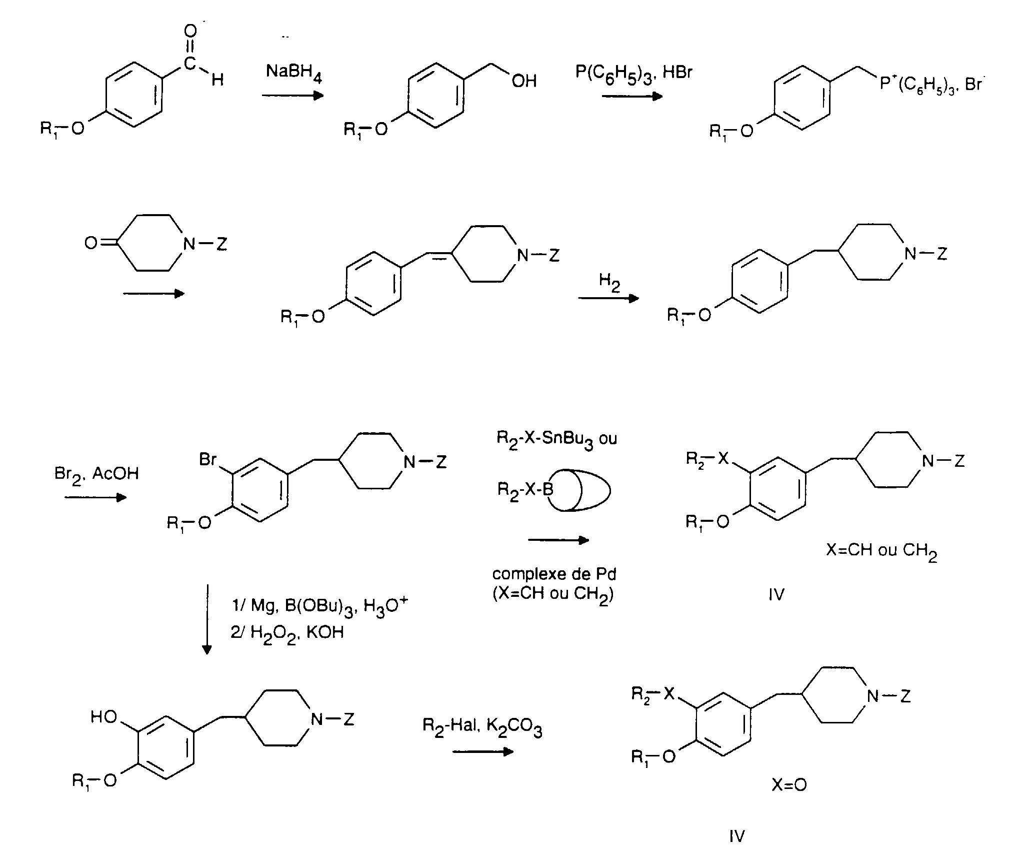

Les benzaldéhydes substitués employés comme matières premières dans le schéma ci-dessus sont des produits commerciaux ou synthétisés par des méthodes connues de l'homme de l'art.

Les méthodes de couplage des bromobenzènes avec les composés organostannés :

(R₂X-SnBu₃)

ou organoborés 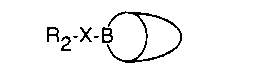 en présence de complexes de palladium et employées dans le schéma ci-dessus sont connues et bien décrites dans la littérature (J. K. STILLE, Angew. Chem. Int. Ed. Engl. 25, 508-524, 1986 ; N. MIYAURA et A. SUZUKI, Chem. Rev., Vol. 95, 2457-2483, 1995).

De plus dans le cas où simultanément X représente un atome d'oxygène, R₂ ne comporte pas de liaison insaturée et R₁ et Z ont les significations précédemment définies, il est particulièrement intéressant d'opérer selon le schéma réactionnel suivant :

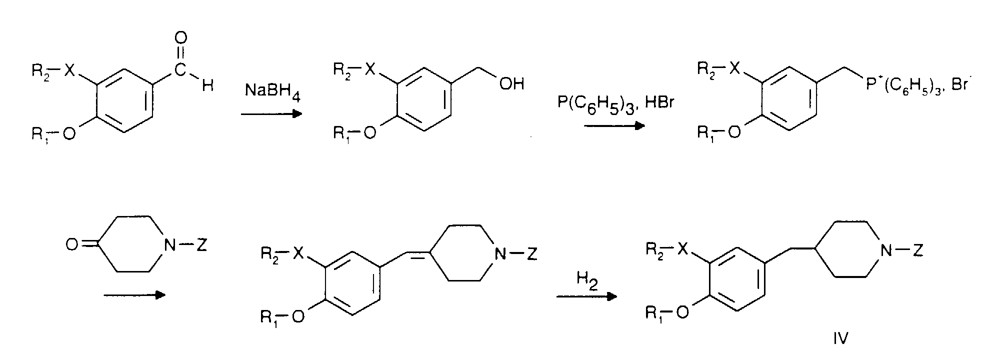

Les benzaldéhydes substitués employés comme produits de départ dans ce dernier schéma ont été synthétisés par les méthodes décrites dans la littérature (N. PALFREYMAN et al., J. Med. Chem., vol 37, pp 1696-1703, 1994).

Les conditions opératoires employées dans les deux schémas précédents pour obtenir les intermédiaires éthyléniques à partir des sels de phosphonium sont celles classiquement décrites pour la réaction de Wittig.

Les composés de la présente invention sont des inhibiteurs très puissants des phosphodiestérases du groupe 4 et de ce fait sont particulièrement intéressants dans les applications thérapeutiques concernant l'inflammation et la relaxation bronchique et plus précisément dans l'asthme et les bronchopathies chroniques obstructives (A. J. DUPLANTIER et J. B. CHENG, Annu. Rep. Med. Chem., vol 29, p 73-81, 1994), (C. D. NICHOLSON et M. SHAHID, Pulmonary Pharmacol., Vol 7, p 1-17, 1994), (T. J. TORPHY, G. P. LIVI et S. B. CHRISTENSEN, Drug News Perspect., Vol 6, p 203-214, 1993), (J. A. LOWE et J. B. CHENG, Drugs Future, Vol 17, p 799-807, 1992), mais aussi dans toutes les affections telles que les rhinites (I. RADERER, E. HAEN, C. SCHUDT et B. PRZYBILLA, Wien. Med. Wochenschr., Vol 145, p 456-458, 1995), le syndrome de détresse respiratoire aigu (SDRA) (C. R. TURNER, K. M. ESSER et E. B. WHEELDON, Circulatory Shock, Vol 39, p 237-245, 1993), les allergies et les dermatites (J. M. HANIFIN et S. C. CHAN, J. Invest. Dermatol., Vol 105, p 84S-88S, 1995), (J. M. HANIFIN, J. Dermatol. Sci., Vol 1, p 1-6, 1990), le psoriasis (E. TOUITOU, N. SHACO-EZRA, N. DAYAN, M. JUSHYNSKI, R. RAFAELOFF et R. AZOURY, J. Pharm. Sci., Vol 81, p 131-134, 1992), (F. LEVI-SCHAFFER et E. TOUITOU, Skin Pharmacol., Vol 4, p 286-290, 1991), l'arthrite rhumatoïde (J. M. ANAYA et L. R. ESPINOZA, J. Rheumatol., Vol 22, p 595-599, 1995), les maladies autoimmunes, (C. P. GENAIN et al. Proc. Natl. Acad. Sci., Vol 92, p 3601-3605, 1995), les scléroses multiples (N. SOMMER et al., Nat. Med., Vol 1, p 244-248, 1995), les dyskinésies (T. KITATANI, S. HAYASHI et T. SAKAGUCHI, Nippon, Yakurigaku, Zasshi, Vol 86, p 353-358, 1985), les glomérulonéphrites (M. HECHT, M. MULLER, M. L. LOHMANN-MATTHES et A. EMMENDORFFER, J. Leukoc. Biol., Vol 57, p 242-249, 1995), l'ostéoarthrite et le choc septique (A. M. BADGER, D. L. OLIVERA et K. M. ESSER Circ. Shock, Vol 44, p 188-195, 1994 ; L. SEKUT et al., Clin. Exp. Immunol., Vol 100, p 126-132, 1995), le sida (T. F. GRETEN, S. ENDRES et al., Aids, Vol 9, p 1137-1144, 1995), la depression (N. A. SACCOMANO et al., J. Med. Chem., Vol 34, p 291-298, 1991), et toute maladie neurodégénérative s'accompagnant de phénomènes inflammatoires comme les maladies d'Alzheimer, de Parkinson, de Huntington, de Down et la sclérose latérale amyotrophique (G. Z. FEUERSTEIN et al., Ann. N. Y. Acad. Sci., Vol 765, p 62-71, 1995).

Ces indications thérapeutiques ne sont pas limitatives dans la mesure où la diminution de la concentration d'AMPc cellulaire, quelles qu'en soient la cause et la localisation tissulaire, aboutit à une disfonction cellulaire, source de phénomènes pathologiques, et peut constituer une cible thérapeutique majeure des produits décrits.

La présente invention a également pour objet les compositions pharmaceutiques contenant comme principe actif un composé de formule générale I ou un de ses sels physiologiquement tolérables, mélangé ou associé à un excipient pharmaceutique approprié, comme par exemple, l'eau distillée, l'amidon, le talc, l'éthylcellulose, le stéarate de magnésium ou un solvant approprié permettant d'inhaler le principe actif sous forme d'aérosol.

Les compositions pharmaceutiques ainsi obtenues se présentent généralement sous forme dosée pouvant contenir de 1 à 500 mg de principe actif, et peuvent revêtir, par exemple, la forme de comprimés, dragées, gélules, suppositoires, solutions injectables ou buvables ou aérosols et être, selon les cas, administrées par voie orale, rectale, parentérale ou locale.

La posologie peut varier selon l'âge et le poids du patient, la voie d'administration, la nature de la maladie et les traitements associés.

A titre d'exemple, la posologie par voie orale peut s'échelonner de 10 à 5 000 mg de principe actif par jour.

Les exemples suivants illustrent la présente invention. Les points de fusion sont, sauf mention contraire, déterminés au tube capillaire.

Dans les procédures de synthèses qui suivent, toutes les matières premières utilisées sont soit disponibles commercialement soit préparées selon des procédés décrits dans la littérature

### EXEMPLE 1 : 4-(3-cyclopentyloxy-4-méthoxybenzyl)-1-(imidazol-4-ylcarbonyl) pipéridine

Une solution contenant 2,1 g de 4-(3-cyclopentyloxy-4-méthoxybenzyl)pipéridine, 2,6 g de tétrafluoroborate de 2-(1H-benzotriazole-1-yl)-1,1,3,3-tétraméthyluronium (TBTU), 1,1 g de N-hydroxybenzotriazole (HOBT), 1,6 ml de diisopropyléthylamine et 1,0 g d'acide 4-imidazole carboxylique, est agitée sous atmosphère d'argon à température ambiante pendant 16 heures.

Le milieu réactionnel est agité quelques minutes avec une solution saturée de carbonate de sodium. La phase organique est ensuite reprise avec de l'eau puis séchée sur sulfate de magnésium et concentrée. L'huile résiduelle est purifiée par chromatographie flash en utilisant un mélange CH₂Cl₂/CH₃OH ammoniacal 95/5 comme système d'élution. La 4-(3-cyclopentyloxy-4-méthoxybenzyl)-1-(imidazol-4-ylcarbonyl)pipéridine, reprise dans l'éthanol chlorhydrique cristallise sous forme de chlorhydrate fondant à 204-207°C.

### EXEMPLES 2 à 71 :

En opérant comme dans l'exemple 1, à partir des matières premières appropriées, ont été préparés les produits objet des exemples suivants :
2) 4-(3-cyclopentyloxy-4-méthoxybenzyl)-1-(1-méthylimidazol-4-ylcarbonyl)pipéridine
3) 1-(5-aminoimidazol-4-ylcarbonyl)-4-(3-cyclopentyloxy-4-méthoxybenzyl) pipéridine
4) 4-(3-cyclopentyloxy-4-méthoxybenzyl)-1-(2-méthylimidazol-4-ylcarbonyl)pipéridine
5) 1-(2-chloro-5-méthylimidazol-4-ylcarbonyl)-4-(3-cyclopentyloxy-4-méthoxybenzyl) pipéridine
6) 1-(2-chloro-1,5-diméthylimidazol-4-ylcarbonyl)-4-(3-cyclopentyloxy-4-méthoxybenzyl) pipéridine
7) 4-(3-cyclopentyloxy-4-méthoxybenzyl)-1-(4-fluorobenzimidazol-2-ylcarbonyl)pipéridine
8) 4-(3-cyclopentylméthyl-4-méthoxybenzyl)-1-(imidazol-4-ylcarbonyl)pipéridine
9) 1-(imidazol-4-ylcarbonyl)-4-(4-méthoxy-3-n-pentyloxybenzyl)pipéridine
10) 1-(imidazol-4-ylcarbonyl)-4-(4-méthoxy-3-n-pentylbenzyl)pipéridine
11) 4-(3-allyloxy-4-méthoxybenzyl)-1-(imidazol-4-ylcarbonyl)pipéridine
12) 4-(3-allyl-4-méthoxybenzyl)-1-(imidazol-4-ylcarbonyl)pipéridine
13) R,S-4-[3-(2-exo-norborn-2-yloxy)-4-méthoxybenzyl]-1-(imidazol-4-ylcarbonyl)pipéridine
14) 4-(3-adamant-1-yloxy-4-méthoxybenzyl-1-(imidazol-4-ylcarbonyl)pipéridine
15) 4-(3-cyclopentyloxy-4-méthoxybenzyl)-1-(pyrrol-2-ylcarbonyl)pipéridine
16) 4-(3-cyclopentylméthyl-4-méthoxybenzyl)-1-(pyrrazol-4-ylcarbonyl)pipéridine
17) 4-(3-cyclopentyloxy-4-méthoxybenzyl)-1-(1-méthylpyrrazol-4-ylcarbonyl)pipéridine
18) 4-(3-cyclopentyloxy-4-méthoxybenzyl)-1-(5-méthylisoxazol-3-ylcarbonyl)pipéridine
19) 4-(3-cyclopentyloxy-4-méthoxybenzyl)-1-(5-n-pentylisoxazol-3-ylcarbonyl)pipéridine
20) 4-(3-cyclopentyloxy-4-méthoxybenzyl)-1-(1,2,3-triazol-4-ylcarbonyl)pipéridine
21) 4-(3-cyclopentyloxy-4-méthoxybenzyl)-1-(1,2,4-triazol-3-ylcarbonyl)pipéridine
22) 4-(3-cyclopentyloxy-4-méthoxybenzyl)-1-(pyrazin-2-ylcarbonyl)pipéridine
23) 4-(4-méthoxy-3-n-pentylbenzyl)-1-(pyrazin-2-ylcarbonyl)pipéridine
24) 1-(5-chloropyrazin-2-ylcarbonyl)-4-(3-cyclopentyloxy-4-méthoxybenzyl)pipéridine
25) 4-(3-cyclopentyloxy-4-méthoxybenzyl)-1-(5-méthylpyrazin-2-ylcarbonyl)pipéridine
26) 1-(6-chloropyridazin-3-ylcarbonyl)-4-(3-cyclopentyloxy-4-méthoxybenzyl)pipéridine
27) 4-(3-cyclopentyloxy-4-méthoxybenzyl)-1-(pyrimid-2-ylcarbonyl)pipéridine
28) 4-(3-cyclopentyloxy-4-méthoxybenzyl)-1-(quinol-4-ylcarbonyl)pipéridine
29) 4-(3-cyclopentyloxy-4-méthoxybenzyl)-1-(quinol-3-ylcarbonyl)pipéridine
30) 4-(3-cyclopentyloxy-4-méthoxybenzyl)-1-(quinol-5-ylcarbonyl)pipéridine
31) 4-(3-cyclopentyloxy-4-méthoxybenzyl)-1-(quinol-6-ylcarbonyl)pipéridine
32) 4-(3-cyclopentyloxy-4-méthoxybenzyl)-1-(quinol-8-ylcarbonyl)pipéridine
33) 4-(3-cyclopentyloxy-4-méthoxybenzyl)-1-(isoquinol-1-ylcarbonyl)pipéridine
34) 4-(3-cyclopentyloxy-4-méthoxybenzyl)-1-(isoquinol-3-ylcarbonyl)pipéridine
35) 4-(3-cyclopentyloxy-4-méthoxybenzyl)-1-(isoquinol-4-ylcarbonyl)pipéridine
36) 4-(3-cyclopentyloxy-4-méthoxybenzyl)-1-(quinoxalin-2-ylcarbonyl)pipéridine
37) 4-(3-cyclopentylméthyl-4-méthoxybenzyl)-1-(quinoxalin-2-ylcarbonyl)pipéridine
38) 4-(4-méthoxy-3-n-pentylbenzyl)-1-(quinoxalin-2-ylcarbonyl)pipéridine
39) 4-(3-cyclopentyloxy-4-méthoxybenzyl)-1-(quinoxalin-6-ylcarbonyl)pipéridine
40) 4-(3-cyclopentyloxy-4-méthoxybenzyl)-1-(indol-2-ylcarbonyl)pipéridine
41) 4-(3-cyclopentyloxy-4-méthoxybenzyl)-1-(5-fluoroindol-2-ylcarbonyl)pipéridine
42) 4-(3-cyclopentyloxy-4-méthoxybenzyl)-1-(indol-3-ylcarbonyl)pipéridine
43) 4-(3-cyclopentyloxy-4-méthoxybenzyl)-1-(1-méthylindol-3-ylcarbonyl)pipéridine
44) 4-(3-cyclopentyloxy-4-méthoxybenzyl)-1-(indol-4-ylcarbonyl)pipéridine
45) 4-(3-cyclopentyloxy-4-méthoxybenzyl)-1-(indol-5-ylcarbonyl)pipéridine
46) 1-(benzimidazol-5-ylcarbonyl)-4-(3-cyclopentyloxy-4-méthoxybenzyl)pipéridine
47) 1-(benzoxazol-5-ylcarbonyl)-4-(3-cyclopentyloxy-4-méthoxybenzyl)pipéridine
48) 4-(3-cyclopentyloxy-4-méthoxybenzyl)-1-(cinnolin-4-ylcarbonyl)pipéridine
49) 4-(3-cyclopentyloxy-4-méthoxybenzyl)-1-(purin-6-ylcarbonyl)pipéridine
50) Iodure de 4-[4-(3-cyclopentyloxy-4-méthoxybenzyl)pipéridin-1-ylcarbonyl]-1-méthylpyridinium
51) Iodure de 3-[4-(3-cyclopentyloxy-4-méthoxybenzyl)pipéridin-1-ylcarbonyl]-1-méthyl pyridinium
52) Chlorhydrate de (R,S)4-[4-méthoxy-3-(5-phényl-2-pentyloxy)benzyl]-1-(imidazol-4-ylcarbonyl)pipéridine
53) 4-[4-Méthoxy-3-(2-méthyl-5-phénylpentyl)benzyl]-1-(imidazol-4-ylcarbonyl)pipéridine
54) 4-(3-Cyclopentyloxy-4-méthoxybenzyl)-1-(4-diméthylaminophénylcarbonyl)pipéridine
55) 1-(2-Chloropyrid-4-ylcarbonyl)-4-(3-cyclopentyloxy-4-méthoxybenzyl)pipéridine
56) 4-(3-Cyclopentyloxy-4-méthoxybenzyl)-1-(4-méthoxycarbonylbenzoyl)pipéridine
57) 1-(4-Carboxybenzoyl)-4-(3-cyclopentyloxy-4-méthoxybenzyl)pipéridine
58) 4-(3-Cyclopentyloxy-4-méthoxybenzyl)-1-(1,2,3-thiadiazol-4-ylcarbonyl)pipéridine
59) 4-(3-Cyclopentyloxy-4-méthoxybenzyl)-1-(3-méthylimidazol-4-ylcarbonyl)pipéridine
60) 4-(3-Cyclopentyloxy-4-méthoxybenzyl)-1-(1,2,5-triméthylpyrrol-3-ylcarbonyl) pipéridine
61) 4-(3-Isopropyloxy-4-méthoxybenzyl)-1-(imidazol-4-ylcarbonyl)pipéridine
62) 6-[4-(3-Cyclopentyloxy-4-méthoxybenzyl)pipéridinocarbonyl]-1*H*-pyrimidine-2,4-dione
63) 5-[4-(3-Cyclopentyloxy-4-méthoxybenzyl)pipéridinocarbonyl]-1*H*-pyrimidine-2,4-dione
64) 4-(3-Butyloxy-4-méthoxybenzyl)-1-[(1-méthylimidazol-4-yl)carbonyl]pipéridine
65) 4-(3-Cyclopentyloxy-4-méthoxybenzyl)-1-(1-isopropylimidazol-4-ylcarbonyl)pipéridine
66) 4-(3-Cyclopentyloxy-4-méthoxybenzyl)-1-(quinol-2-ylcarbonyl)pipéridine
67) 4-(3-Cyclopentyloxy-4-méthoxybenzyl)-1-(pyrazol-4-ylcarbonyl)pipéridine
68) 4-(3-Cyclopentyloxy-4-méthoxybenzyl)-1-(2,6-dichlorobenzoyl)pipéridine
69) 4-(3-Cyclopentyloxy-4-méthoxybenzyl)-1-(2,5-diméthylpyrrol-3-ylcarbonyl)pipéridine
70) 1-(4-Chlorobenzoyl)-4-(3-cyclopentyloxy-4-méthoxybenzyl)pipéridine
71) 4-(3-Cyclopentyloxy-4-difluorométhoxybenzyl)-1-(3-méthylimidazol-4-ylcarbonyl) pipéridine

### EXEMPLE 72 :4-(3-cyclopentyloxy-4-méthoxybenzyl)-1-(4-méthoxybenzoyl)pipéridine

Une solution de 20 ml de tétrahydrofurane (THF) contenant 1 g de chlorure de 4-méthoxybenzoyle est ajoutée goutte à goutte dans une solution de 30 ml de THF contenant 0,73 g de 4-(3-cyclopentyloxy-4-méthoxybenzyl)pipéridine, 0,78 ml de triéthylamine et 0,2 g de 4-N,N-diméthylaminopyridine et agitée sous argon à température ambiante. Le milieu réactionnel est agité 4 heures à température ambiante, dilué avec 150 ml d'éther éthylique, lavé avec une solution aqueuse saturée de carbonate de sodium, puis avec de l'eau, lavé avec une solution d'HCI 1N, puis lavé à nouveau avec de l'eau. La phase organique est séchée sur sulfate de magnésium et concentrée. L'huile résiduelle est filtrée sur silice par technique de chromatographie flash avec un mélange toluène/éthanol 97/3 utilisé comme éluant. La 4-(3-cyclopentyloxy-4-méthoxybenzyl)-1-(4-méthoxybenzoyl)pipéridine est obtenue sous forme d'huile.

### EXEMPLES 73-96

En opérant comme dans l'exemple 73, à partir des matières premières appropriées, ont été préparés les produits objet des exemples suivants :
73) 4-(3-cyclopentyloxy-4-méthoxybenzyl)-1-(3,4-diméthoxybenzoyl)pipéridine
74) 4-(3-cyclopentyloxy-4-méthoxybenzyl)-1-(3,4,5-triméthoxybenzoyl)pipéridine
75) 1-(4-acétyloxybenzoyl)-4-(3-cyclopentyloxy-4-méthoxybenzyl)pipéridine
76) 1-(4-aminosulfonylbenzoyl)-4-(3-cyclopentyloxy-4-méthoxybenzyl)pipéridine
77) 4-(3-cyclopentyloxy-4-méthoxybenzyl)-1-(4-trifluorométhylbenzoyl)pipéridine
78) 4-(3-cyclopentyloxy-4-méthoxybenzyl)-1-(2-nitrobenzoyl)pipéridine
79) 4-(3-cyclopentyloxy-4-méthoxybenzyl)-1-(3-nitrobenzoyl)pipéridine
80) 4-(3-cyclopentyloxy-4-méthoxybenzyl)-1-(4-nitrobenzoyl)pipéridine
81) 4-(3-cyclopentyloxy-4-méthoxybenzyl)-1-(thién-2-ylcarbonyl)pipéridine
82) 4-(3-cyclopentyloxy-4-méthoxybenzyl)-1-(furan-2-ylcarbonyl)pipéridine
83) 4-(3-cyclopentyloxy-4-méthoxybenzyl)-1-(furan-3-ylcarbonyl)pipéridine
84) 4-(3-cyclopentyloxy-4-méthoxybenzyl)-1-nicotinoylpipéridine
85) 1-(5-acétamidonicotinoyl)-4-(3-cyclopentyloxy-4-méthoxybenzyl)pipéridine
86) 1-(6-acétamidonicotinoyl)-4-(3-cyclopentyloxy-4-méthoxybenzyl)pipéridine
87) 4-(3-cyclopentyloxy-4-méthoxybenzyl)-1-(6-hydroxynicotinoyl)pipéridine
88) 4-(3-cyclopentyloxy-4-méthoxybenzyl)-1-picolinoylpipéridine
89) 1-(2-chloro-6-méthoxypyrid-4-ylcarbonyl)-4-(3-cydopentyloxy-4-méthoxybenzyl) pipéridine
90) 4-(3-cyclopentyloxy-4-méthoxybenzyl)-1-isonicotinoylpipéridine
91) 1-(2-Chloronicotinoyl)-4-(3-cyclopentyloxy-4-méthoxybenzyl)pipéridine
92) 1-(6-Chloronicotinoyl)-4-(3-cyclopentyloxy-4-méthoxybenzyl)pipéridine
93) 4-(3-Cyclopentyloxy-4-méthoxybenzyl)-1-(2,6-dichloroisonicotinoyl)pipéridine
94) 1-(2-Chloro-4-trifluorométhylnicotinoyl)-4-(3-cyclopentyloxy-4-méthoxybenzyl)pipéridine
95) 4-(3-Cyclopentyloxy-4-méthoxybenzyl)-1-(2,5-dichloronicotinoyl)pipéridine
96) 1-(2-Chloro-6-méthylisonicotinoyl)-4-(3-cyclopentyloxy-4-méthoxybenzyl)pipéridine

### EXEMPLE 97 : ETUDE PHARMACOLOGIQUE

### Mesure de l'activité PDE

Les cellules U937 sont cultivées dans un milieu de culture (RPMI) contenant 10% de sérum de veau foetal. Brièvement, les cellules sont lysées puis ensuite centrifugées (100000 g, 60 min., 4°C) et le surnageant est récupéré en vue d'une séparation par HPLC des différentes formes de PDE (C. Lugnier et V.B. Schini, Biochem. Pharmacol., vol 39 p75-84, 1990).

L'activité PDE est mesurée par l'apparition de [³H]5' AMP résultant de l'hydrolyse du [³H]AMP cyclique. La PDE et le [³H]AMP cyclique (1µCi/ml) sont incubés 30 minutes à 30°C. La radioactivité est mesurée au moyen d'un compteur à scintillation liquide (Beckman LS 1701).

La PDE 4 est caractérisée par :
- l'hydrolyse de l'AMP cyclique
- l'absence d'inhibition par le GMP cyclique de l'hydrolyse d'AMP cyclique
- l'inhibition par le rolipram, molécule de référence.

Les composés sont étudiés à deux concentrations (10⁻⁷M et 10⁻⁵M) en duplicate. Les résultats sont exprimés en % d'inhibition de l'activité phosphodiestérasique.

Les composés de la présente invention présentent une inhibition très importante de l'activité phosphodiestérasique, inhibition qui, par exemple, peut dépasser 80%, dès la concentration de 10⁻⁷M.

## Revendications

1. Les amines cycliques N-substituées de formule I : 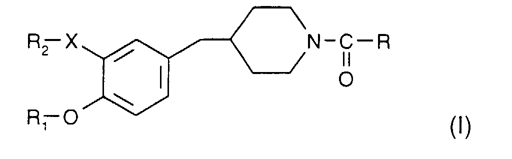 dans laquelle :
- X est choisi parmi -CH=, -CH₂- et un atome d'oxygène ;
- R₁ représente un radical choisi parmi le groupe constitué par les radicaux (C₁-C₆)alkyle, en chaîne droite et ramifiée, non substitués et substitués par un et plusieurs atomes d'halogène ;
- R₂ représente un radical choisi parmi le groupe constitué par :
a) les radicaux hydrocarbonés monocycliques saturés et non-saturés contenant de 3 à 6 atomes de carbone non substitués et ces mêmes radicaux substitués par un et plusieurs substituants choisis parmi le groupe constitué par : les atomes d'halogène et le radical hydroxy ;
b) les radicaux hydrocarbonés polycycliques saturés et non-saturés contenant de 7 à 10 atomes de carbone non-substitués et ces mêmes radicaux substitués par un et plusieurs substituants choisis parmi le groupe constitué par : les atomes d'halogène et le radical hydroxy ;
c) les radicaux (C₁-C₁₃) hydrocarbonés saturés et non saturés en chaîne droite et en chaîne ramifiée, non-substitués et substitués par un et plusieurs substituants choisis parmi les atomes d'halogène et le radical hydroxy ; et
d) les radicaux (C₁-C₁₃)hydrocarbonés définis au paragraphe c) ci-dessus, substitués de plus par un et plusieurs radicaux choisis parmi le groupe constitué par:
• le radical phényle non substitué et les radicaux phényles substitués par un et plusieurs substituants choisis parmi le groupe constitué par les atomes d'halogène et le radical hydroxy ;
• les radicaux hydrocarbonés monocycliques saturés et non-saturés contenant de 3 à 6 atomes de carbone non substitués et ces mêmes radicaux substitués par un et plusieurs substituants choisis parmi le groupe constitué par : les atomes d'halogène et le radical hydroxy ;
• les radicaux hydrocarbonés polycycliques, saturés et non-saturés, contenant de 7 à 10 atomes de carbone non-substitués et ces mêmes radicaux substitués par un et plusieurs substituants choisis parmi le groupe constitué par : les atomes d'halogène et le radical hydroxy ;
- R est choisi parmi le groupe constitué par
- les radicaux phényle, biphénylyle et naphtyle ;
- les radicaux aromatiques, comportant 5 chaînons et contenant de 1 à 4 hétéroatomes identiques et différents ;
- les radicaux aromatiques, comportant 6 chaînons et contenant 1 à 3 atomes d'azote.
- les radicaux aromatiques bicycliques saturés et non-saturés, comportant de 9 à 10 atomes dont 1 à 4 hétéroatomes identiques et différents ;
- les radicaux aromatiques bicycliques partiellement saturés ci-dessus définis contenant une et plusieurs fonctions carbonylées ;
et
- chacun des groupements précédemment définis entrant dans la signification de R, substitués par 1 à 3 substituants choisis parmi les atomes d'halogène, les radicaux R₁, -O-CO-R₁, (R₁ ayant la signification précédemment définie), CN, NO₂, OR₃, SR₃, COR₃, COOR₃, NR₃R₄, NCOR₃, CONR₃R₄, SO₂NR₃R₄ dans lesquels, R₃ et R₄ identiques ou différents représentent chacun un atome d'hydrogène ou R₁ tel que précédemment défini.
leurs isomères géométriques et leurs isomères optiques,
ainsi que leurs sels d'addition avec des acides ou des bases pharmaceutiquement acceptables.

2. Un composé de la revendication 1 qui est la 4-(3-cyclopentyloxy-4-méthoxybenzyl)-1-(1-méthylimidazol-4-ylcarbonyl)pipéridine.

3. Un composé de la revendication 1 qui est la 4-(3-cyclopentyloxy-4-méthoxybenzyl)-1-nicotinoyl pipéridine.

4. Un composé de la revendication 1 qui est la 4-(3-cyclopentyloxy-4-méthoxybenzyl)-1-(quinol-4-ylcarbonyl)pipéridine.

5. Un composé de la revendication 1 qui est la 4-(3-cyclopentyloxy-4-méthoxybenzyl)-1-(quinoxalin-2-ylcarbonyl)pipéridine.

6. Le procédé de préparation des composés de la revendication 1 caractérisé en ce que l'on fait réagir :
- l'amine secondaire cyclique de formule II : 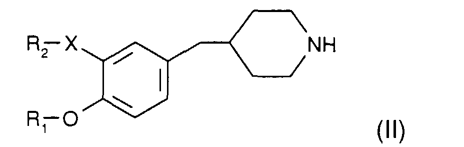 dans laquelle : X, R₁ et R₂ ont les significations définies dans la revendication 1,
avec
- le composé carbonylé de formule III : 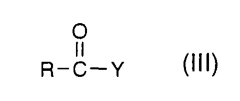 dans laquelle :
- R a la signification définie dans la revendication 1 et
- Y représente un radical hydroxy ou un atome de chlore.

7. Les compositions pharmaceutiques, agissant comme inhibiteur des phosphodiestérases du groupe 4, contenant comme principe actif un composé selon l'une quelconque des revendications 1 à 5, avec un ou plusieurs excipients pharmaceutiques appropriés.

## Claims

1. N-substituted cyclic amines of formula I : 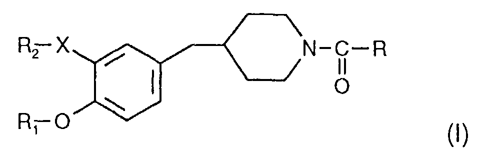 wherein :
- X is selected from -CH=, -CH₂- and an oxygen atom ;
- R₁ represents a radical selected from the group consisting of straight-chain and branched (C₁-C₆)alkyl radicals that are unsusbstituted or substituted by one or more halogen atoms ;
- R2 represents a radical selected from the group consisting of:
a) saturated and unsaturated monocyclic hydrocarbon radicals having from 3 to 6 carbon atoms and being unsubstituted, and those same radicals substituted by one or more substituents selected from the group consisting of : halogen atoms and the hydroxy radical;
b) saturated and unsaturated polycyclic hydrocarbon radicals having from 7 to 10 carbon atoms and being unsubstituted, and those same radicals substituted by one or more substituents selected from the group consisting of : halogen atoms and the hydroxy radical;
c) saturated and unsaturated, straight-chain and branched (C₁-C₁₃)hydrocarbon radicals that are unsubstituted, and those same radicals substituted by one or more substituents selected from halogen atoms and the hydroxy radical; and
d) the (C₁-C₁₃)hydrocarbon radicals defined in paragraph c) hereinabove, substituted in addition by one or more radicals selected from the group consisting of:
• the unsubstituted phenyl radical, and phenyl radicals substituted by one or more substituents selected from the group consisting of halogen atoms and the hydroxy radical;
• saturated and unsaturated monocyclic hydrocarbon radicals having from 3 to 6 carbon atoms and being unsubstituted, and those same radicals substituted by one or more substituents selected from the group consisting of : halogen atoms and the hydroxy radical;
• saturated and unsaturated polycyclic hydrocarbon radicals having from 7 to 10 carbon atoms and being unsubstituted, and those same radicals substituted by one or more substituents selected from the group consisting of : halogen atoms and the hydroxy radical;
- R is selected from the group consisting of
- the radicals phenyl, biphenylyl and naphthyl ;
- aromatic radicals having 5 ring members and containing from 1 to 4 identical or different hetero atoms;
- aromatic radicals having 6 ring members and containing from 1 to 3 nitrogen atoms;
- saturated and unsaturated bicyclic aromatic radicals having from 9 to 10 atoms including from 1 to 4 identical or different hetero atoms ;
- the partially saturated bicyclic aromatic radicals defined hereinabove, containing one or more carbonyl functions ;
and
- each of the groups defined hereinabove for R, substituted by from 1 to 3 substituents selected from halogen atoms and the radicals R₁, -O-CO-R₁ (R₁ being as defined above), CN, NO₂, OR₃, SR₃, COR₃, COOR₃, NR₃R₄, NCOR₃, CONR₃R₄ and SO₂NR₃R₄, with R₃ and R₄, which are identical or different, each representing a hydrogen atom or R₁ as defined hereinabove ;
their geometric isomers and their optical isomers,
and addition salts thereof with pharmaceutically acceptable acids or bases.

2. A compound of claim 1, which is 4-(3-cyclopentyloxy-4-methoxybenzyl)-1-(1-methylimidazol-4-ylcarbonyl)piperidine.

3. A compound of claim 1, which is 4-(3-cyclopentyloxy-4-methoxybenzyl)-1-nicotinoylpiperidine.

4. A compound of claim 1, which is 4-(3-cyclopentyloxy-4-methoxybenzyl)-1-(quinol-4-ylcarbonyl)piperidine.

5. A compound of claim 1, which is 4-(3-cyclopentyloxy-4-methoxybenzyl)-1-(quinoxalin-2-ylcarbonyl)piperidine.

6. A process for the preparation of the compounds of claim 1, characterised in that:
- a cyclic secondary amine of formula II : 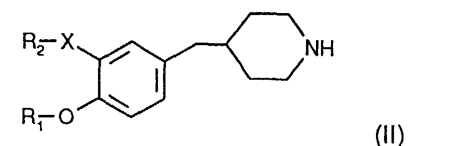 wherein : X, R₁ and R₂ are as defined in claim 1,
is reacted with
- a carbonyl compound of formula III : 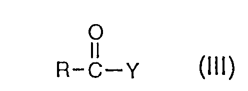 wherein :
- R is as defined in claim 1 and
- Y represents a hydroxy radical or a chlorine atom.

7. Pharmaceutical compositions, acting as inhibitor of group 4 phosphodiesterases, comprising as active ingredient a compound according to any one of claims 1 to 5, together with one or more appropriate pharmaceutical excipients.

## Patentansprüche

1. Cyclische N-substituierte Amine der Formel I: 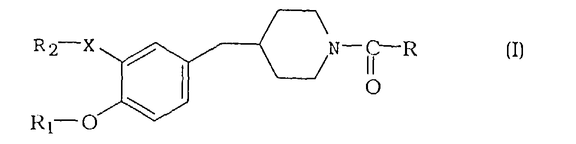 in der:
- X ausgewählt ist aus -CH=, -CH₂- und einem Sauerstoffatom;
- R₁ eine Gruppe bedeutet ausgewählt aus der Gruppe, die durch (C₁-C₆)-Alkyl-gruppen mit gerader oder verzweigter Kette, die nicht substituiert oder durch ein oder mehrere Halogenatome substituiert sind, gebildet ist;
- R₂ eine Gruppe bedeutet ausgewählt aus der Gruppe, die gebildet ist durch:
a) gesättigte und ungesättigte monocyclische Kohlenwasserstoffreste, die 3 bis 6 Kohlenstoffatome enthalten, die nicht substituiert sind, und die gleichen Reste, die durch einen oder mehrere Substituenten ausgewählt aus der durch Halogen-atome und Hydroxygruppen gebildeten Gruppe substituiert sind;
b) gesättigte und ungesättigte polycyclische Kohlenwasserstoffreste, die 7 bis 10 Kohlenstoffatome enthalten, die nicht substituiert sind, und die gleichen Reste, die durch einen oder mehrere Substituenten ausgewählt aus der Halogenatome und Hydroxygruppen gebildeten Gruppe substituiert sind;
c) gesättigte und ungesättigte (C₁-C₁₃)-Kohlenwasserstoffreste mit gerader oder verzweigter Kette, die nicht substituiert sind und die durch einen oder mehrere Substituenten ausgewählt aus Halogenatomen und Hydroxygruppen substituiert sind; und
d) die in dem obigen Absatz c) definierten (C₁-C₁₃)-Kohlenwasserstoffreste, die zusätzlich durch einen oder mehrere Reste substituiert sind ausgewählt aus der Gruppe, die gebildet wird durch:
• nichtsubstituierte Phenylreste und Phenylreste, die durch einen oder mehrere Substituenten ausgewählt aus der Halogenatome und Hydroxygruppen umfassenden Gruppe substituiert sind;
• gesättigte und ungesättigte monocyclische Kohlenwasserstoffreste, die 3 bis 6 Kohlenstoffatome enthalten und nicht substituiert sind, und die gleichen Reste, die durch einen oder mehrere Substituenten ausgewählt aus der durch Halogenatome und Hydroxygruppen gebildeten Gruppe substituiert sind;
• gesättigte und ungesättigte polycyclische Kohlenwasserstoffreste, die 7 bis 10 Kohlenstoffatome enthalten und nicht substituiert sind, und die gleichen Reste, die durch einen oder mehrere Substituenten ausgewählt aus der durch Halogenatome und Hydroxygruppen gebildeten Gruppe substituiert sind;
- R ausgewählt ist aus der Gruppe, die gebildet wird durch:
- Phenyl-, Biphenylyl- und Naphthylreste;
- aromatische Reste mit 5 Kettengliedern, die 1 bis 4 gleichartige oder verschiedene Heteroatome enthalten;
- aromatische Reste mit 6 Kettengliedern, die 1 bis 3 Stickstoffatome enthalten;
- gesättigte und ungesättigte bicyclische aromatische Reste, die 9 bis 10 Atome aufweisen, darunter 1 bis 4 gleichartige oder verschiedene Heteroatome;
- teilweise gesättigte bicyclische aromatische Reste, wie sie oben definiert worden sind, die eine oder mehrere Carbonylfunktionen enthalten; und
- jede der oben für die Bedeutung von R definierten Gruppen, die durch 1 bis 3 Substituenten substituiert sind, ausgewählt aus Halogenatomen, Gruppen R₁, -O-CO-R₁ (worin R₁ die oben angegebenen Bedeutungen besitzt), CN, NO₂, OR₃, SR₃, COR₃, COOR₃, NR₃R₄, NCOR₃, CONR₃R₄, SO₂NR₃R₄, worin R₃ und R₄, die gleichartig oder verschieden sein können, jeweils ein Wasserstoffatom oder R₁, wie es oben definiert worden ist, bedeuten,
deren geometrische Isomere und deren optische Isomere,
sowie deren Additionssalze mit pharmazeutisch annehmbaren Säuren oder Basen.

2. Verbindung nach Anspruch 1, nämlich 4-(3-Cyclopentyloxy-4-methoxybenzyl)-1-(1-methylimidazol-4-yl-carbonyl)-piperidin.

3. Verbindung nach Anspruch 1, nämlich 4-(3-Cyclopentyloxy-4-methoxybenzyl)-1-nicotinoyl-piperidin.

4. Verbindung nach Anspruch 1, nämlich 4-(3-Cyclopentyloxy-4-methoxybenzyl)-1-(chinol-4-yl-carbonyl)-piperidin.

5. Verbindung nach Anspruch 1, nämlich 4-(3-Cyclopentyloxy-4-methoxybenzyl)-1-(chinoxalin-2-yl-carbonyl)-piperidin.

6. Verfahren zur Herstellung der Verbindungen nach Anspruch 1,
dadurch gekennzeichnet, daß man
- ein cyclisches sekundäres Amin der Formel II: 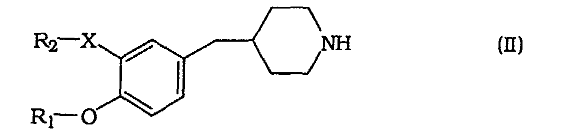 in der X, R₁ und R₂ die in Anspruch 1 angegebenen Bedeutungen besitzen, mit
- einer Carbonylverbindung der Formel III: 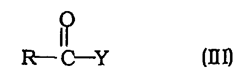 in der:
- R die in Anspruch 1 angegebenen Bedeutungen besitzt und
- Y eine Hydroxygruppe oder ein Chloratom darstellt,
umsetzt.

7. Pharmazeutische Zubereitungen, die als Inhibitoren der Phosphodiesterasen der Gruppe 4 wirksam sind, enthaltend als Wirkstoff eine Verbindung nach einem der Ansprüche 1 bis 5 zusammen mit einem oder mehreren pharmazeutisch geeigneten Trägermaterialien.
